(19) Europäisches Patentamt
European Patent Office
Office européen des brevets

(11) **EP 2 493 515 B1**

(12) **FASCICULE DE BREVET EUROPEEN**

(45) Date de publication et mention
de la délivrance du brevet:
**27.06.2018 Bulletin 2018/26**

(21) Numéro de dépôt: **10787852.2**

(22) Date de dépôt: **26.10.2010**

(51) Int Cl.:
*A61L 2/10* (2006.01)     *B08B 9/00* (2006.01)
*B65B 55/00* (2006.01)    *B65B 55/08* (2006.01)

(86) Numéro de dépôt international:
**PCT/FR2010/052291**

(87) Numéro de publication internationale:
**WO 2011/051615 (05.05.2011 Gazette 2011/18)**

(54) **DISPOSITIF DE TRAITEMENT PAR LUMIERE PULSEE REFROIDIE**

BEHANDLUNGSVORRICHTUNG MIT GEKÜHLTEM PULSLICHT

COOLED PULSED LIGHT TREATMENT DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**

(30) Priorité: **30.10.2009  FR 0957679**

(43) Date de publication de la demande:
**05.09.2012  Bulletin 2012/36**

(73) Titulaire: **Claranor
84140 Montfavet (FR)**

(72) Inventeur: **HOUDE, Eric
F-13860 Peyrolles En Provence (FR)**

(74) Mandataire: **Pontet Allano & Associes
Parc Les Algorithmes, Bâtiment Platon
CS 70003 Saint-Aubin
91192 Gif-sur-Yvette Cedex (FR)**

(56) Documents cités:
**WO-A2-00/38740          WO-A2-02/26270
US-A- 4 910 942          US-A- 6 054 097
US-A1- 2006 228 251**

## Description

## Domaine technique

La présente invention concerne un dispositif de traitement en vue de décontamination par lumière puisée. Elle concerne aussi un appareil comprenant un tel dispositif.

Le domaine de l'invention est plus particulièrement mais de manière non limitative celui du conditionnement et de l'emballage de produits alimentaires et médicaux.

## Etat de la technique antérieure

Le document US 2006 / 0228251 décrit un appareil de stérilisation par pulse de lumière haute intensité au moyen d'une lampe, cet appareil comprenant un ventilateur pour générer une circulation de fluide dans l'enceinte de la lampe.

Le principe de la décontamination et de la stérilisation par exposition à des impulsions ou flashs de lumière ultraviolette est connu de longue date.

Cette technique exploite l'efficacité bactéricide des rayons ultraviolets contenue dans des flashs intenses de lumière blanche.

L'efficacité décontaminante de la lumière pulsée a été démontrée sur une large gamme de microorganismes : bactéries, moisissures, virus, etc. La part de rayonnement UV (entre 200 et 300 nm) comprise dans le flash entraîne, par la combinaison d'un effet photothermique et d'une réaction photochimique, la destruction des microorganismes présents sur le produit soumis au traitement.

L'effet photochimique résulte de l'absorption des rayons UV par l'ADN des microorganismes. Cette absorption présente des maxima autour de 200 nm et de 257 nm, respectivement. Elle provoque des ruptures et la formation de liaisons anormales au sein des brins des molécules d'ADN, qui empêchent leur réplication.

L'effet photothermique est dû au fait que les rayonnements absorbés par les micro-organismes provoquent une augmentation brutale de température. L'absorption de l'énergie reçue provoque la rupture des membranes cellulaires et la destruction des micro-organismes. La durée de ce pic de température, fonction de la durée de l'impulsion lumineuse, peut être extrêmement courte, typiquement de l'ordre de quelques centaines de microsecondes. L'élévation de température (jusqu'à 150°C) a lieu au niveau microscopique, au niveau macroscopique la température du produit traité n'augmente pratiquement pas.

Selon de nombreux travaux tels que ceux de Wuytack et al. (Wuytack, E.Y., Thi Phuong, L.D., Aertsen, A., Reyns, K.M.F., Marquenie, D., De Ketelaere, B., et al., "Comparison of sublethal injury induced in Salmonella enterica serovar Typhimurium by heat and by différent non thermal treatments", Journal of Food Protection, 2003, vol. 66, pp. 1071-1073), c'est l'association des deux effets, photochimique et photothermique, qui expliquerait l'efficacité du traitement par lumière puisée.

De manière habituelle, les impulsions de lumière sont générées au moyens de lampes flash au Xénon. Les lampes flash sont des lampes à arc fonctionnant en mode pulsé. L'énergie électrique est accumulée dans un condensateur électrique. Un signal à haute tension (plusieurs dizaines de kV) déclenche l'amorçage d'un arc électrique dans le gaz contenu dans la lampe. La libération de l'énergie électrique produit à son tour par ionisation du gaz une émission lumineuse. Le xénon est le gaz inerte le plus efficace pour la conversion de l'énergie électrique en énergie lumineuse, en particulier dans l'UV. Pour les pressions de gaz utilisées (égales ou supérieures à 1 bar), le spectre des flashs est continu. Il est constitué de longueurs d'onde allant de l'UV au proche infrarouge (200 à 1100 nm). La durée de ces flashs est typiquement de l'ordre de la centaine de microsecondes ou plus.

Le niveau de décontamination obtenu est dépendant du nombre et de la puissance des flashs appliqués, de la nature de la surface traitée, et va de la simple réduction logarithmique (destruction d'une fraction des microorganismes) à la stérilisation complète (destruction quasiment totale des microorganismes).

On connaît le document US 4,910,942 de Dunn et al. dans lequel les auteurs détaillent les conditions de mise en oeuvre de la technique de décontamination par flashs de lumière pulsée pour des applications dans l'industrie agroalimentaire et pharmaceutique : emballages d'aliments, emballages de médicaments, films plastiques, liquides, aliments (poissons, fromage, pâtisseries). Les auteurs détaillent également l'effet décontaminant obtenu dans différents cas en fonction du nombre de flashs et de l'énergie (en joules) par flash.

La technique de décontamination par flashs de lumière pulsée est un procédé athermique au niveau macroscopique, dans le sens où les mécanismes physiques mis en oeuvre (effets photochimique et photothermique) sont macroscopiquement essentiellement athermiques, et donc n'entraînent pas en eux-mêmes d'élévation sensible de la température moyenne des objets décontaminés. Cette propriété est importante pour le traitement des d'objets sensibles à la chaleur, et en particulier la conservation des propriétés organoleptiques des aliments.

Toutefois, lors de la mise en oeuvre pratique de la technique de décontamination par flashs de lumière pulsée, l'échauffement s'avère en général un problème important qui entraîne des limitations gênantes. Cet échauffement provient de l'absorption de l'énergie lumineuse par les objets à décontaminer, mais également et surtout par les parties de la machine exposées au rayonnement en permanence et qui sont en contact avec ces objets traités. L'effet thermique du rayonnement est d'autant plus important que les lampes flash au xénon utilisées pour des raisons pratiques et économiques émettent plus de 85% de leur densité spectrale d'énergie dans la partie visible et infrarouge du spectre lumineux.

Les effets thermiques sont en général limités dans les dispositifs de l'art antérieur d'une part en limitant les cadences d'illumination, donc les cadences de traitement des objets, et d'autre part en utilisant les lampes avec des niveaux d'énergie d'excitation qui permettent d'optimiser la part spectrale de rayonnement UV, au prix toutefois d'une réduction importante de leur durée de vie.

Le but de la présente invention est de proposer un dispositif de traitement par lumière pulsée refroidie, qui permette des cadences de traitement élevées compatibles avec les besoins d'une utilisation en ligne de production, d'objets sensibles à l'échauffement, tout en optimisant les coûts d'exploitation.

**Exposé de l'invention**

Cet objectif est atteint avec un dispositif à haute cadence de décontamination d'objets par lumière pulsée comprenant :

- des moyens d'illumination permettant d'illuminer une zone de décontamination dans laquelle sont disposés les objets, lesquels moyens d'illumination sont aptes à produire des trains d'impulsions de lumière comprenant des longueurs d'ondes comprises entre 200 nm et 300 nm, selon une cadence réglable d'impulsion,
- des moyens de positionnement aptes à positionner les objets dans la zone de décontamination selon une disposition prédéterminée,
- des moyens de transfert aptes à faire défiler les objets dans la zone de décontamination selon une cadence réglable de défilement, la cadence d'impulsion étant réglée de telle sorte que chaque objet reçoive dans ladite zone de décontamination un nombre prédéterminé d'impulsions de lumière,

caractérisé en ce qu'il comprend en outre des moyens de refroidissement permettant de maintenir la température de la zone de décontamination sensiblement en dessous d'une température prédéterminée lorsque ladite zone de décontamination est illuminée, lesquels moyens de refroidissement comprennent des moyens de circulation d'un fluide de refroidissement intégrés dans les moyens de positionnement.

Les moyens de positionnement sont conformés de telle sorte que :

- ils comprennent une gorge, de telle sorte à permettre le guidage des objets à décontaminer par coulissement, et
- au moins une partie des parois de ladite gorge est en contact thermique avec au moins une partie des parois des moyens de circulation de fluide de refroidissement.

Les moyens de d'illumination peuvent comprendre :

- une source de lumière comprenant une lampe flash, laquelle lampe flash pouvant contenir par exemple du xénon,
- un réflecteur métallique pourvu d'une surface apte à concentrer par réflexion la lumière issue de ladite source de lumière dans la zone de décontamination.

Avantageusement, les moyens de refroidissement peuvent comprendre en outre :

- des moyens de circulation d'un fluide de refroidissement intégrés dans le réflecteur,
- des moyens de circulation d'un fluide de refroidissement disposés autour de la lampe flash, lesquels moyens pouvant comprendre un tube en quartz.

Les moyens de circulation de fluide de refroidissement peuvent avantageusement être alimentés par le même circuit de fluide de refroidissement, c'est-à-dire constituer des parties d'un circuit unique disposés en parallèle et/ou en série.

Le fluide de refroidissement peut avantageusement comprendre de l'eau désionisée.

Suivant d'autres caractéristiques avantageuses,

- le dispositif suivant l'invention peut comprendre en outre une fenêtre sensiblement transparente dans au moins une partie du spectre de la lumière émise, laquelle fenêtre est disposée sur le trajet de la lumière entre la surface du réflecteur d'une part, et la zone de décontamination d'autre part,
- la fenêtre transparente peut être en quartz,
- la fenêtre sensiblement transparente peut être fixée sur le réflecteur,
- les moyens de refroidissement peuvent comprendre en outre des moyens d'injection d'air permettant de faire circuler de d'air sous pression dans un espace délimité par le réflecteur et la face intérieure de ladite fenêtre transparente,
- la lampe, le réflecteur, les moyens de positionnement et la zone de décontamination peuvent s'étendre sensiblement selon une direction de défilement, et les moyens de circulation de fluide de refroidissement peuvent comprendre des tubes sensiblement rectilignes, disposés sensiblement selon ladite direction de défilement,
- les moyens de refroidissement, notamment les tubes, peuvent être constitués de matériaux n'altérant pas l'eau désionisée, tels que de l'inox (ou acier inoxydable),

Suivant un mode de réalisation, le dispositif suivant l'invention peut avantageusement comprendre en outre au moins un rétroréflecteur sensiblement diffusant, disposé selon au moins une des parois de la zone de décontamination. Ce ou ces rétroréflecteurs peuvent par exemple permettre d'illuminer dans de meilleures conditions certaines zones lorsque les objets s'y prêtent. On peut citer notamment le cas d'objets pourvus de plusieurs ouvertures, auquel cas il devient possible d'illuminer des zones externes des objets à la périphérie de ces ouvertures. Les rétroréflecteurs peuvent être réalisés par exemple par collage d'un support rétroréfléchissant, dépôt de peinture, ou traitement (polissage, ...) de la paroi de la zone de décontamination.

Suivant une réalisation avantageuse du dispositif selon l'invention dans lequel la lampe mise en oeuvre est une lampe flash au xénon, le circuit de contrôle de la lampe peut être configuré de telle sorte que l'énergie électrique injectée dans la lampe à chaque impulsion soit déterminée de telle sorte que la durée de vie attendue de la lampe soit supérieure à dix millions d'impulsions.

Cela revient à limiter l'énergie électrique injectée dans la lampe pour augmenter sa durée de vie. Bien entendu, cette limitation n'est effectuée que dans la limite de la nécessité de conserver une intensité lumineuse dans la gamme des UV-C compatible avec les besoins de la décontamination. Ce mode de fonctionnement permet de diminuer les coûts de maintenance du système de décontamination à haute cadence, mais il est défavorable du point de vue thermique. Il est rendu possible dans le dispositif selon l'invention par les moyens de refroidissements mis en oeuvre.

En effet, le spectre d'une telle lampe flash au xénon s'étend de l'infrarouge à l'ultraviolet. On sait que la partie ultraviolette et plus spécifiquement la gamme dite des UV-C, c'est-à-dire des longueurs d'onde comprises entre 200 nm et 300 nm sont indispensables à la décontamination. Cependant on ne sait pas obtenir des lampes à Xénon ayant un bon rendement dans la gamme des UV-C sans pénaliser leur durée de vie. En effet, la durée de vie (V) d'une lampe à arc au xénon est reliée à l'énergie appliquée (E) par la relation suivante :

$$V \sim E^{-8.5}.$$

Par exemple, une lampe recevant 300 Joules en 200 $\mu$s et réémettant 150 Joules dont 10 % dans la partie UV-C du spectre a une durée de vie de l'ordre de 3 millions de flashs. En doublant l'énergie injectée, la proportion spectrale dans la gamme des UV-C passe à 14 % mais la durée de vie de la lampe est ramenée à 8000 flashs. C'est pourquoi, dans des conditions d'utilisation permettant une durée de vie raisonnable, de l'ordre de 90 % de l'émission spectrale de la lampe se situe dans la partie visible ou infrarouge du spectre et n'a pour effet que d'apporter de la chaleur.

La présente invention concerne également un appareil de décontamination par lumière pulsée intégrant le dispositif, caractérisé en ce que qu'il est mis en oeuvre en ligne de production.

Un tel appareil peut être mis en oeuvre par exemple pour décontaminer les objets suivants :

- des bouchons de bouteille,
- des bouchons de bouteille présentant une seconde ouverture à l'opposé de l'ouverture apte à se fixer sur la bouteille, appelés communément bouchons « sport »,
- des préformes de bouteilles, en PET notamment.

## Description des figures et modes de réalisation

D'autres avantages et particularités de l'invention apparaîtront à la lecture de la description détaillée de mises en oeuvre et de modes de réalisation nullement limitatifs, et des dessins annexés suivants :

- la figure 1 illustre un premier mode de réalisation de dispositif selon l'invention, permettant de décontaminer des bouchons de bouteille,
- la figure 2 illustre un deuxième mode de réalisation de dispositif selon l'invention, permettant de décontaminer des bouchons de bouteille « sport »,
- la figure 3 présente une vue d'un appareil selon l'invention.

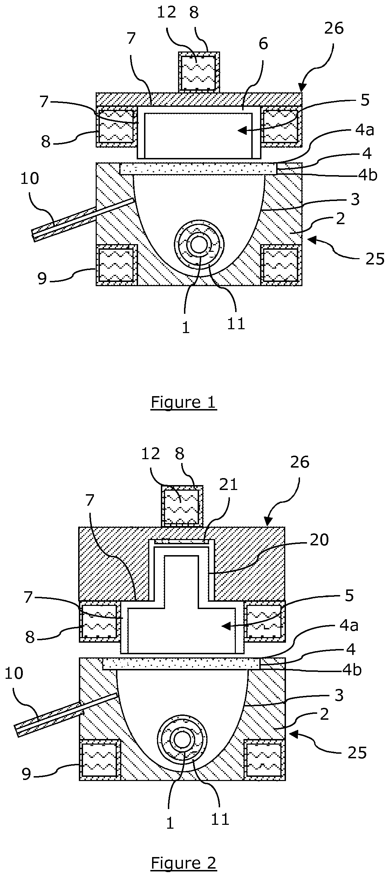

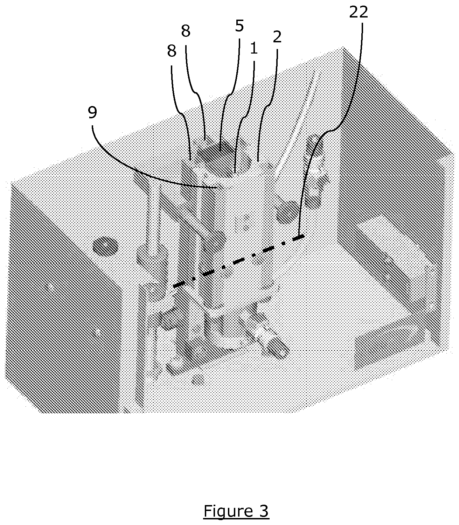

En référence à la figure 1, le dispositif selon l'invention est avantageusement mis en oeuvre dans un appareil de décontamination de bouchons de bouteilles à destination de l'industrie alimentaire. Cet appareil peut être notamment installé en ligne de production pour décontaminer les bouchons avant le remplissage et la fermeture des bouteilles. Les bouchons sont par exemple en PE (polyéthylène) ou en PP (polypropylène), et les bouteilles en en PET (polyéthylène téréphtalate). Une vue en perspective du même dispositif est présenté à la figure 3. La figure 1 correspond à une vue en coupe du dispositif selon l'axe 22.

Le dispositif comporte une cavité optique constituée d'un réflecteur 2 fermé par une fenêtre transparente 4 de préférence en quartz, et d'une lampe flash au xénon 1 de forme tubulaire. La lampe 1 est elle-même insérée à l'intérieur d'un tube en quartz 11 dans lequel circule le liquide de refroidissement 12 qui est de préférence, mais de manière nullement limitative, de l'eau désionisée. Le réflecteur 2 est de préférence en aluminium poli et il est refroidi par des tubes de section sensiblement carrée 9 de préférence en acier inoxydables plaqués sur le corps du réflecteur 2 et dans lesquels circule également le liquide de refroidissement.

La forme de la surface 3 du réflecteur 2 est calculée pour illuminer de façon optimale la zone de décontamination 5. En face de la cavité se trouvent les guides 7 de circulation des objets à décontaminer 6, tels que des bouchons. Ces guides 7 constituent une gorge de forme sensiblement adaptée à celle des objets 6. Ils sont accolés à des tubes de section sensiblement carrée 8 de préférence en acier inoxydable refroidis eux aussi par une circulation du liquide de refroidissement. Avantageusement, au moins une partie des tubes de refroidissement 8 sont directement utilisés pour guider les objets 6, de telle sorte à optimiser les échanges thermiques. La fenêtre 4 participe également au guidage des objets 6.

Une ligne de conditionnement de bouteilles peut fonctionner jusqu'à une cadence de l'ordre de 72000 bouteilles par heure. Le dispositif selon l'invention, lorsqu'il est inséré dans cette ligne, doit donc pouvoir traiter 20 bouchons par secondes. Dans un dispositif tel que celui présenté à la figure 3, la zone de décontamination 5 a une longueur telle que quatre bouchons peuvent être traités simultanément. La cadence d'émission des impulsions lumineuses nécessaire pour que chaque bouchon reçoive au moins un flash est donc au minimum de 5 Hz, pour une décontamination de 3 logs (c'est-à-dire une réduction d'un facteur 1000 du nombre de microorganismes présents après traitement).

Les lampes utilisées de manière habituelle pour traiter des bouchons émettent une puissance moyenne optique importante, par exemple de l'ordre de 750 W pour des impulsions de 150 Joules émises à 5 Hz. L'échauffement des bouchons 6 demeure modéré car ils ne reçoivent qu'un ou deux flashs. Par contre les différentes pièces de la machine telles que les guides 7 et réflecteurs 2, qui sont immobiles, reçoivent en permanence cette puissance et sont susceptibles de s'échauffer fortement. Selon leur nature, aluminium ou acier inoxydable par exemple, ces pièces peuvent absorber entre 10 et 40 % du rayonnement incident. De ce fait, si aucune précaution particulière n'est prise, lorsque les bouchons 6 en matière plastique entrent en contact avec ces pièces très chaudes, à 100°C ou plus, ils risquent de fondre voire même de s'enflammer. Il peut en résulter un blocage des bouchons, un arrêt de la production et une détérioration de la machine. Ainsi, les moyens de refroidissement tels que les tubes 8 intégrés aux éléments de guidage 7, qui sont un objet de la présente invention, constituent des éléments indispensable à la mise en oeuvre du dispositif dans la mesure où ils permettent l'évacuation de la chaleur des parties statiques de la zone de décontamination 5, de telle sorte que les surfaces environnant les objets 6 ne dépassent pas une température maximale, par exemple de 100°C.

De même, l'échauffement du réflecteur 2 doit être évité car les bouchons 6 circulent au contact de la fenêtre 4 qui ferme la cavité optique. Si cette cavité s'échauffe cela augmente la température de la fenêtre 4, ce qui peut aussi provoquer la fusion des bouchons. Le contrôle de la température cette fenêtre 4 est avantageusement assurée dans le dispositif selon l'invention par les moyens de refroidissement tels que les tubes 9 dans le réflecteur 2 et le tube 11 autour de la lampe 1.

Lorsque la puissance dissipée dans le dispositif est importante, par exemple lorsqu'il est mis en oeuvre deux lampes fonctionnant à 5 Hz et dissipant une puissance totale de 1500 W, la fenêtre 4 peut également avantageusement être refroidie par injection d'air filtré 10 sur sa face 4b située du côté de la lampe 1.

En référence à la figure 2, suivant un deuxième mode de réalisation, lorsque les objets 20 sont partiellement transparents ou présentent des ouvertures, un ou plusieurs rétrodiffuseurs 21 peuvent être disposés sur les guides 7 de telle sorte à rétrodiffuser la lumière sur les faces qui ne sont pas directement illuminées. En particulier, un dispositif selon l'invention permet avantageusement de décontaminer des bouchons de bouteille « sports » 20, lesquels sont pourvus d'une seconde ouverture à l'opposé de la partie qui se fixe sur la bouteille, qui permet de boire sans les dévisser. Le rétroréflecteur 21 tel que présenté à la figure 2 est illuminé au travers de cette seconde ouverture et renvoie la lumière de telle sorte à illuminer et décontaminer les rebords extérieurs du bouchon 20 autour de cette seconde ouverture.

Suivant des modes de réalisation particuliers :

- Tous les éléments dans lesquels circule le liquide de refroidissement peuvent être reliés au même circuit ;
- Dans un dispositif de forme essentiellement allongée comme présenté à la figure 3, les moyens de refroidissement 8, 9, 11 peuvent être disposés en parallèle par rapport à la circulation du liquide ;
- Il est possible d'utiliser tout type de liquide de refroidissement sans sortir du champ de l'invention ;
- La source 1 peut comprendre tout type de sources compatibles avec l'application. En particulier, elle peut être une lampe à eximères ou un laser ;
- La fenêtre 4 peut comporter un traitement, ou comprendre un matériau tel qu'elle ne laisse passer qu'une partie du spectre optique de la source 1. En particulier la fenêtre 4 peut être une lame dichroïque bloquant le rayonnement infrarouge qui est à l'origine d'une part importante de l'effet thermique ;

Un dispositif selon l'invention peut bien entendu être mis en oeuvre pour la décontamination de tous types d'objets, parmi lesquels on peut également citer à titre d'exemples non limitatifs des opercules et des pots. Un dispositif selon l'invention peut également avantageusement être mis en oeuvre pour la décontamination d'objets de dimension supérieure à la zone de décontamination et défilant dans celle-ci sur des supports sensiblement immobiles, tels que par exemple des films plastiques ou constitués d'autres matériaux.

Bien sûr, l'invention n'est pas limitée aux exemples qui viennent d'être décrits et de nombreux aménagements peuvent être apportés à ces exemples sans sortir du cadre de l'invention.

**Revendications**

1. Dispositif à haute cadence de décontamination d'objets par lumière pulsée comprenant :

- des moyens d'illumination (25) permettant d'illuminer une zone de décontamination (5) dans laquelle sont disposés les objets (6), lesquels moyens d'illumination sont aptes à produire des trains d'impulsions de lumière comprenant des longueurs d'ondes comprises entre 200 nm et 300 nm selon une cadence réglable d'impulsion,
- des moyens de positionnement (26) aptes à positionner les objets (6) dans la zone de décontamination (5) selon une disposition prédéterminée,
- des moyens de transfert aptes à faire défiler les objets dans la zone de décontamination selon une cadence réglable de défilement, la cadence d'impulsion étant réglée de telle sorte que chaque objet (6) reçoive dans ladite zone de décontamination un nombre prédéterminé d'impulsions de lumière,

**caractérisé en ce qu'**il comprend en outre des moyens de refroidissement permettant de maintenir la température de la zone de décontamination (5) en dessous d'une température prédéterminée lorsque ladite zone de décontamination est illuminée, lesquels moyens de refroidissement comprennent des premiers moyens de circulation (8) d'un fluide de refroidissement (12) intégrés dans les moyens de positionnement (26), les moyens de positionnement (26) comprenant une gorge, de telle sorte à permettre le guidage des objets à décontaminer par coulissement, au moins une partie des parois (7) de ladite gorge étant en contact thermique avec au moins une partie des parois des premiers moyens de circulation de fluide de refroidissement (8).

2. Dispositif selon l'une des revendications 1, **caractérisé en ce que** les moyens d'illumination (25) comprennent :

- une source de lumière (1) comprenant une lampe flash,
- un réflecteur métallique (2) pourvu d'une surface (3) apte à concentrer par réflexion la lumière issue de ladite source de lumière (1) dans la zone de décontamination (5).

3. Dispositif selon la revendication 2, **caractérisé en ce que** les moyens de refroidissement comprennent en outre des seconds moyens de circulation (9) d'un fluide de refroidissement (12) intégrés dans le réflecteur (2).

4. Dispositif selon l'une des revendications 2 ou 3, **caractérisé en ce que** les moyens de refroidissement comprennent en outre des troisièmes moyens de circulation (11) d'un fluide de refroidissement (12) disposés autour de la lampe flash (1).

5. Dispositif selon l'une quelconque des revendications précédentes, **caractérisé en ce que** les moyens de circulation de fluide de refroidissement sont alimentés par le même circuit de fluide de refroidissement (12).

6. Dispositif selon l'une quelconque des revendications précédentes, **caractérisé en ce que** le fluide de refroidissement (12) comprend de l'eau désionisée.

7. Dispositif selon l'une quelconque des revendications 2 à 6, **caractérisé en ce qu'**il comprend en outre une fenêtre transparente (4) dans au moins une partie du spectre de la lumière émise, laquelle fenêtre est disposée sur le trajet de la lumière entre la surface du réflecteur (3) d'une part, et la zone de décontamination (5) d'autre part.

8. Dispositif selon la revendication 7, **caractérisé en ce que** la fenêtre transparente (4) est fixée sur le réflecteur (2) et **en ce que** les moyens de refroidissement comprennent en outre des moyens d'injection d'air (10) permettant de faire circuler de d'air sous pression dans un espace délimité par le réflecteur et la face intérieure de ladite fenêtre transparente (4).

9. Dispositif selon l'une quelconque des revendications 2 à 8, **caractérisé en ce que**

- la lampe (1), le réflecteur (2), les moyens de positionnement (26) et la zone de décontamination (5) s'étendent selon une direction de défilement, et
- les moyens de circulation de fluide de refroidissement (8, 9) comprennent des tubes rectilignes, disposés selon ladite direction de défilement.

10. Dispositif selon l'une quelconque des revendications précédentes, **caractérisé en ce qu'**il comprend en outre au moins un rétroréflecteur (21) diffusant, disposé selon au moins une des parois (7) de la zone de décontamination (5).

11. Dispositif selon l'une quelconque des revendications 2 à 10, **caractérisé en ce que** l'énergie électrique injectée dans la lampe (1) à chaque impulsion est déterminée de telle sorte que la durée de vie attendue de la lampe (1) soit supérieure à dix millions d'impulsions.

12. Appareil de décontamination par lumière pulsée comprenant un dispositif selon l'une quelconque des revendications 1 à 11, **caractérisé en ce que** les objets décontaminés (6) sont des bouchons de bou-

teile.

13. Appareil de décontamination par lumière pulsée comprenant un dispositif selon l'une quelconque des revendications 1 à 11, **caractérisé en ce que** les objets décontaminés sont des bouchons (20) de bouteille « sports » qui présentent une seconde ouverture à l'opposé de l'ouverture apte à se fixer sur la bouteille.

14. Appareil de décontamination par lumière pulsée comprenant un dispositif selon l'une quelconque des revendications 1 à 11, **caractérisé en ce que** les objets décontaminés sont des préformes de bouteilles.

**Patentansprüche**

1. Vorrichtung zum Dekontaminieren von Objekten durch gepulstes Licht mit hoher Arbeitsrate, enthaltend:

   - Beleuchtungseinrichtungen (25), mit denen ein Dekontaminierungsbereich (5) beleuchtet werden kann, in welchem die Objekte (6) angeordnet sind, wobei die Beleuchtungseinrichtungen dazu geeignet sind, Lichtpulsfolgen mit Wellenlängen zwischen 200 nm und 300 nm nach einer einstellbaren Pulsrate zu erzeugen,
   - Positionierungseinrichtungen (26), die dazu geeignet sind, die Objekte (6) in dem Dekontaminierungsbereich (5) gemäß einer vorbestimmten Anordnung zu positionieren,
   - Überführungseinrichtungen, die dazu geeignet sind, die Objekte in dem Dekontaminierungsbereich mit einer einstellbaren Durchlaufrate durchlaufen zu lassen, wobei die Pulsrate so eingestellt wird, dass jedes Objekt (6) in dem Dekontaminierungsbereich eine vorbestimmte Anzahl von Lichtpulsen empfängt,

   **dadurch gekennzeichnet, dass** sie ferner Kühleinrichtungen enthält, die ermöglichen, die Temperatur des Dekontaminierungsbereichs (5) unter einer vorbestimmten Temperatur zu halten, wenn der Dekontaminierungsbereich beleuchtet wird, wobei die Kühleinrichtungen erste Zirkulationseinrichtungen (8) zum Zirkulieren von einem Kühlfluid (12) enthalten, die in den Positionierungseinrichtungen (26) integriert sind, wobei die Positionierungseinrichtungen (26) eine Nut aufweisen, so dass das Führen der zu dekontaminierenden Objekte durch Gleitbewegung möglich ist, wobei zumindest ein Teil der Wände (7) der Nut in Wärmekontakt mit zumindest einem Teil der Wände der ersten Zirkulationseinrichtungen zur Zirkulation von Kühlfluid (8) ist.

2. Vorrichtung nach Anspruch 1, **dadurch gekennzeichnet, dass** die Beleuchtungseinrichtungen (25) enthalten:

   - eine Lichtquelle (1) mit einer Blitzlampe,
   - einen metallischen Reflektor (2), der mit einer Fläche (3) versehen ist, die dazu geeignet ist, das von der Lichtquelle (1) stammende Licht durch Reflexion in den Dekontaminierungsbereich (5) zu konzentrieren.

3. Vorrichtung nach Anspruch 2, **dadurch gekennzeichnet, dass** die Kühleinrichtungen ferner zweite Zirkulationseinrichtungen (9) zur Zirkulation von Kühlfluid (12) enthalten, die in dem Reflektor (2) integriert sind.

4. Vorrichtung nach einem der Ansprüche 2 oder 3, **dadurch gekennzeichnet, dass** die Kühleinrichtungen ferner dritte Zirkulationseinrichtungen (11) zur Zirkulation von Kühlfluid (12) enthalten, die um die Blitzlampe (1) herum angeordnet sind.

5. Vorrichtung nach einem der vorangehenden Ansprüche, **dadurch gekennzeichnet, dass** die Zirkulationseinrichtungen zur Zirkulation von Kühlfluid über den gleichen Kühlfluidkreislauf (12) versorgt werden.

6. Vorrichtung nach einem der vorangehenden Ansprüche, **dadurch gekennzeichnet, dass** das Kühlfluid (12) entionisiertes Wasser enthält.

7. Vorrichtung nach einem der Ansprüche 2 bis 6, **dadurch gekennzeichnet, dass** sie ferner ein in zumindest einem Teil des Spektrums des emittierten Lichts transparentes Fenster (4) aufweist, welches Fenster im Strahlengang zwischen der Oberfläche des Reflektors (3) einerseits und dem Dekontaminierungsbereich (5) andererseits angeordnet ist.

8. Vorrichtung nach Anspruch 7, **dadurch gekennzeichnet, dass** das transparente Fenster (4) an den Reflektor (2) befestigt ist und dass die Kühleinrichtungen ferner Lufteinleiteinrichtungen (10) aufweisen, mit denen Druckluft in einem Raum umgewälzt werden kann, der von dem Reflektor und der Innenseite des transparenten Fensters (4) begrenzt wird.

9. Vorrichtung nach einem der Ansprüche 2 bis 8, **dadurch gekennzeichnet, dass**

   - die Lampe (1), der Reflektor (2), die Positionierungseinrichtungen (26) und der Dekontaminierungsbereich (5) sich in einer Durchlaufrichtung erstrecken, und
   - die Zirkulationseinrichtungen zur Zirkulation von Kühlfluid (8, 9) geradlinige Röhren enthalten, die in der Durchlaufrichtung angeordnet sind.

10. Vorrichtung nach einem der vorangehenden Ansprüche, **dadurch gekennzeichnet, dass** sie ferner zumindest einen streuenden Retroreflektor (21) enthält, der entlang zumindest einer der Wände (7) des Dekontaminierungsbereichs (5) angeordnet ist.

11. Vorrichtung nach einem der Ansprüche 2 bis 10, **dadurch gekennzeichnet, dass** die bei jedem Puls in die Lampe (1) eingeleitete elektrische Energie so bestimmt ist, dass die erwartete Lebensdauer der Lampe (1) über zehn Millionen Pulse beträgt.

12. Dekontaminierungsgerät zur Dekontaminierung durch gepulstes Licht, enthaltend eine Vorrichtung nach einem der Ansprüche 1 bis 11, **dadurch gekennzeichnet, dass** die dekontaminierten Objekte (6) Flaschenstopfen sind.

13. Dekontaminierungsgerät zur Dekontaminierung durch gepulstes Licht, enthaltend eine Vorrichtung nach einem der Ansprüche 1 bis 11, **dadurch gekennzeichnet, dass** die dekontaminierten Objekte sogenannte "Sport"-Flaschenstopfen (20) sind, die eine zweite Öffnung entgegengesetzt zur Öffnung aufweisen, die an die Flasche fixiert werden kann.

14. Dekontaminierungsgerät zur Dekontaminierung durch gepulstes Licht, enthaltend eine Vorrichtung nach einem der Ansprüche 1 bis 11, **dadurch gekennzeichnet, dass** die dekontaminierten Objekte Flaschenvorformen sind.

**Claims**

1. A device for the high-speed decontamination of objects by means of pulsed light, including:

    - an illuminating means (25) for illuminating a decontamination area (5) in which the objects (6) are arranged, which illuminating means is suitable for producing streams of light pulses having wavelengths comprised between 200 nm to 300 nm according to an adjustable pulse rate,
    - a positioning means (26) suitable for positioning the objects (6) in the decontamination area (5) according to a predetermined arrangement,
    - a transfer means suitable for scrolling the objects in the decontamination area according to an adjustable scrolling rate, the pulse rate being adjusted such that each object (6) receives in said decontamination area a predetermined number of light pulses,

    **characterised in that** it further includes a cooling means for maintaining the temperature of the decontamination area (5) below a predetermined temperature when said decontamination area is illuminated, which cooling means includes a first means (8) for circulating a cooling fluid (12) built into the positioning means (26), the positioning means (26) including a throat, in order to allow slidably guiding the objects (6) to be decontaminated, at least one part of the walls (7) of said throat thermally contacting at least one part of the walls of the first means for circulating the cooling fluid (8).

2. The device according to claim 1, **characterised in that** the illuminating means (25) includes:

    - a light source (1) comprising a flash lamp,
    - a metal reflector (2) provided with a surface (3) suitable for concentrating through reflection the light from said light source (1) into the decontamination area (5).

3. The device according to claim 2, **characterised in that** the cooling means further includes a second means (9) for circulating a cooling fluid (12) built into the reflector (2).

4. The device according to one of claims 2 or 3, **characterised in that** the cooling means further includes a third means (11) for circulating a cooling fluid (12) arranged about the flash lamp (1).

5. The device according to any of the preceding claims, **characterised in that** the means for circulating the cooling fluid is fed by the same cooling fluid circuit (12).

6. The device according to any of the preceding claims, **characterised in that** the cooling fluid (12) includes deionised water.

7. The device according to any of claims 2 to 6, **characterised in that** it further includes a transparent window (4) in at least one part of the spectrum of the emitted light, which window is arranged on the light path between the surface of the reflector (3) on the one hand, and the decontamination area (5) on the other hand.

8. The device according to claim 7, **characterised in that** the transparent window (4) is attached to the reflector (2) and **in that** the cooling means further includes an air injecting means (10) for circulating pressurized air into a space bounded by the reflector and the inner face of said transparent window (4).

9. The device according to any of claims 2 to 8, **characterised in that**

- the lamp (1), the reflector (2), the positioning means (26) and the decontamination area (5) extend along a scrolling direction, and
- the means for circulating the cooling fluid (8, 9) includes rectilinear tubes, arranged along said scrolling direction.

10. The device according to any of the preceding claims, **characterised in that** it further includes at least one scattering retroreflector (21), arranged along at least one of the walls (7) of the decontamination area (5).

11. The device according to any of claims 2 to 10, **characterised in that** the electrical energy injected into the lamp (1) at each pulse is determined such that the life expectancy of the lamp (1) is higher than ten million pulses.

12. A pulsed light decontamination appliance including a device according to any of the preceding claims 1 to 11, **characterised in that** the decontaminated objects (6) are bottle caps.

13. The pulsed light decontamination appliance including a device according to any of the preceding claims 1 to 11, **characterised in that** the decontaminated objects are "sport" bottle caps (20) which have a second opening opposite to the opening suitable for being attached to the bottle.

14. The pulsed light decontamination appliance including a device according to any of the preceding claims 1 to 11, **characterised in that** the decontaminated objects are bottle preforms.

Figure 1

Figure 2

Figure 3

## RÉFÉRENCES CITÉES DANS LA DESCRIPTION

**Documents brevets cités dans la description**

- US 20060228251 A

- US 4910942 A, Dunn

**Littérature non-brevet citée dans la description**

- **WUYTACK, E.Y. ; THI PHUONG, L.D. ; AERTSEN, A. ; REYNS, K.M.F. ; MARQUENIE, D. ; DE KETELAERE, B. et al.** Comparison of sublethal injury induced in Salmonella enterica serovar Typhimurium by heat and by différent non thermal treatments. *Journal of Food Protection,* 2003, vol. 66, 1071-1073